# EUROPEAN PATENT APPLICATION

(11) **EP 2 009 045 A1**
(43) Date of publication of application: **31.12.2008**
(21) Application number: 08011441.6
(22) Date of filing: 24.06.2008
(51) Int. Cl.: C08K 3/34, C08K 9/02, C09C 1/30, C08J 5/00

(54) **Method for manufacturing thermosetting resin composite material**

(30) Priority: 25.06.2007 JP 2007165820
(71) Applicant: Akebono Brake Industry CO., LTD., Tokyo 103-8534 (JP)
(72) Inventor: Aoyagi, Yoshihiro, Tokyo (JP); Idei, Hiroshi, Tokyo (JP); Kurihara, Shou, Tokyo (JP); Okumura, Naeko, Tokyo (JP)
(74) Representative: Grünecker, Kinkeldey, Stockmair & Schwanhäusser Anwaltssozietät

(57) **Abstract**

A thermosetting resin composite material is manufactured by forming a polybenzoxazine resin by polycondensation reaction of a compound having a phenolic hydroxyl group, primary amines, and formaldehydes in the presence of a swelling clay mineral having been subjected to organizing treatment, or by adding the swelling clay mineral to the reaction system after termination of the polycondensation reaction. A friction material is provided with the thermosetting resin composite material obtained by the above method.

## Description

This application claims foreign priority from Japanese Patent Application No. 2007-165820 filed on June 25, 2007, the entire contents of which are hereby incorporated by reference.

### BACKGROUND OF THE INVENTION

### <FIELD OF THE INVENTION>

The present invention relates to a method for manufacturing a thermosetting resin composite material, a thermosetting resin composite material obtained by the same method, and a friction material using the same composite material. More specifically, the invention relates to a method for manufacturing a thermosetting resin composite material in which a swelling clay mineral such as montmorillonite is homogeneously dispersed and improved in strength and heat resistance, a thermosetting resin composite material obtained by the same method, and a friction material using the same composite material and improved in performance at high temperature.

### <BACKGROUND ART>

A polybenzoxazine resin shows excellent heat resistance as compared with a phenol resin and an epoxy resin, and which resin does not generate by-products at the time of molding, so that attracting public attention as a novel material. However, further heat resistance is required of friction materials, and improvement in frictional characteristics at high temperature is expected by using resins excellent in heat resistance.

Dispersion of organized clay minerals in resins has been examined hitherto for the purpose of the improvements in heat resistance and mechanical strength of various resins (forexample, refer to Patent Documents 1, 2 and 3).
[Patent Document 1] JP-A-09-194822
[Patent Document 2] JP-A-10-330534
[Patent Document 3] JP-A-2002-212386

For organizing laminar (swellable) clay minerals, for example, quaternary ammonium salts and amines are used. For dispersing an organized laminar clay mineral in a resin, a method of mixing a manufactured resin and an organized laminar clay mineral in a solvent, or a method of mechanically mixing a resin and an organized laminar clay mineral with a biaxial kneader is generally used. However, great shear force is required for achieving homogeneous dispersion with these methods, so that industrially unsuitable.

### SUMMARY OF THE INVENTION

One or more embodiments of the invention provide a manufacturing method of a thermosetting resin composite material in which a swelling clay mineral such as montmorillonite is homogeneously dispersed and strength and heat resistance are improved. In addition, one or more embodiments of the invention provide a thermosetting resin composite material obtained by the method, and a friction material comprising the composite material improved in performances at high temperature.

According to a first aspect of the invention, a method for manufacturing a thermosetting resin composite material is provided with: forming a polybenzoxazine resin by polycondensation reaction of a compound having a phenolic hydroxyl group, primary amines, and formaldehydes in the presence of a swelling clay mineral having been subjected to organizing treatment, or adding the swelling clay mineral to the reaction system after termination of the polycondensation reaction.

According to a second aspect of the invention, in the method of the first aspect, the swelling clay mineral having been subjected to organizing treatment may be montmorillonite having been subjected to organizing treatment.

According to a third aspect of the invention, in the method of the first or second aspect, the swelling clay mineral having been subjected to organizing treatment may be a one having been subjected to the treatment with at least one of amines and quaternary ammonium salt.

According to a fourth aspect of the invention, in the method of the third aspect, the amines may be aromatic amines.

According to a fifth aspect of the invention, in the method of any one of first to fourth aspects, the primary amines to be used as the raw material of the polybenzoxazine resin may be primary aromatic amines.

According to sixth aspect of the invention, a thermosetting resin composite material is obtained by the method of any one of first to fifth aspects.

According to a seventh aspect of the invention, a friction material is provided with the thermosetting resin composite material of the sixth aspect.

In accordance with one or more embodiments, a method for manufacturing a thermosetting resin composite material in which a swelling clay mineral such as montmorillonite is homogeneously dispersed and strength and heat resistance are improved, a thermosetting resin composite material obtained by the same method, and a friction material comprising the same composite material improved in performances at high temperature can be provided.

Other aspects and advantages of the invention will be apparent from the following description, the drawings and the claims.

### BRIEF DESCRIPTION OF THE DRAWINGS

Fig. 1 is an X-ray diffraction chart of montmorillonite having been subjected to organizing treatment and untreated montmorillonite.
Fig. 2 is an X-ray diffraction chart showing the results of evaluation of widening of the distance between layers by montmorillonite in the thermosetting resin composite materials in Examples 5 and 6 and Comparative Examples 5.
Fig. 3 is a microphotograph showing the state of dispersion of the montmorillonite in the thermosetting resin composite material in Example 5.

### DETAILED DESCRIPTION OF THE EXEMPLARY EMBODIMENTS

According to an exemplary embodiment of the invention, in a method for manufacturing a thermosetting resin composite material, apolybenzoxazine resin is formed by a polycondensation reaction of a compound having a phenolic hydroxyl group, primary amines, and formaldehydes in the presence of a swelling clay mineral having been subjected to organizing treatment, or by adding the swelling clay mineral to the reaction system after termination of the polycondensation reaction.

### Swelling clay mineral having been subjected to organizing treatment:

In the method according to the embodiment, a swelling clay mineral having been subjected to organizing treatment is used. As the swelling claymineral to be treated, for example, smectitesseries clay mineralssuch asmontmorillonite,saponite, beidellite, nontronite, hectorite, and stevensite, vermiculite and halloysite are exemplified, and these swelling clayminerals may be natural products or may be synthesized products. Of these minerals, montmorillonite is especially preferred from the viewpoints that treatment is easy and the effect of improvement of reinforcement as a filler is great.

These swelling clay minerals have a laminar structure, and organizing treatment forms an interlaminar compound and, at the same time, the distance between layers is widened, and layer separation is liable to occur. As the organic compounds for use in the organizing treatment, amines and quaternary ammonium salts are exemplified. The examples of the amines that may be used include aliphatic amines and aromatic amines having from 1 to 18 carbon atoms. The specific examples of the aliphatic amines include hydrochloride and bromate of diethylamine, amylamine, dodecylamine, stearylamine, and didodecylmethylamine, and the specific examples of the aromatic amines include aniline, toluidine, xylidine, and phenylenediamine. Of these amines, aniline is especially preferred. On the other hand, as the quaternary ammonium salts, e.g., dimethyldioctadecylammonium chloride, and oleyl-bis(2-hydroxyethyl)methylammonium chloride may be preferably exemplified.

### Raw materials for manufacturing polybenzoxazine resin:

In the method of the embodiment, as the raw materials for manufacturing a polybenzoxazine resin, a compound having a phenolic hydroxyl group, primary amines, and formaldehydes are used.

As the compound having a phenolichydroxyl group, monohydric, or dihydric or higher polyhydric phenols having a hydrogen atom at at least either one ortho-position of the hydroxyl group on an aromatic ring may be used, specifically monohydric phenols, such as phenol, o-cresol, m-cresol, p-cresol, xylenol, p-t-butylphenol, α-naphthol, β-naphthol, and p-phenylphenol; dihydric phenols, such as catechol, resorcinol, 4,4'-dihydroxydiphenylmethane (bisphenol F), and 2, 2-bis(4-hydroxyphenyl)propane (bisphenol A); and trihydric or higher polyhydric phenols such as a trisphenol compound, a tetraphenol compound, and a phenol resin may be exemplified. Of these compounds, bisphenol A is preferred from the viewpoint of the performances of the polybenzoxazine resin to be obtained.

On the other hand, there are aliphatic amines and aromatic amines as the primary amines, but when aliphatic amines are used, the polybenzoxazine resin to be obtained is inferior in heat resistance, and aromatic amines are preferred. As the aromatic amines, e.g., aniline, toluidine, xylidine and anisidine may be exemplified.

As the formaldehydes, formalin, paraformaldehyde and trioxan may be exemplified.

### Condensation reaction:

In the method of the embodiment, a thermosetting resin composite material may be manufactured by polycondensation reaction of the raw materials of a polybenzoxazine resin, that is, a compound having a phenolic hydroxyl group, primary amines, and formaldehydes to form a polybenzoxazine resin, in the presence of a swelling clay mineral having been subjected to organizing treatment.

The condensation reaction is preferably performed in the proportion of primary amines of from 0.5 to 1.0 mol and the like per mol of all the phenolic hydroxyl groups, and preferably from 0.6 to 1.0 mol, and formaldehyde of preferably 2 mols or more per mol of the primary amines.

The reaction may be carried out by heating treatment of the compound having a phenolic hydroxyl group, primary amines and formaldehydes in an appropriate solvent, e.g., water, lower alcohol such as methanol or ethanol, or ketones such as methyl ethyl ketone or methyl isobutyl ketone at temperature of from 40 to 90°C or so in the presence of a swelling clay mineral having been subjected to organizing treatment. A thermosetting resin composite material containing a polybenzoxazine resin and homogeneously dispersed swelling clay mineral having been subjected to organizing treatment may be obtained by solid-liquid separation and drying after termination of the reaction, or by distilling the solvent under reduced pressure.

Further, in the method of the embodiment, a thermosetting resin composite material containing a polybenzoxazine resin and homogeneously dispersed swelling clay mineral having been subjected to organizing treatment may also be obtained in the absence of a swelling clay mineral having been subjected to organizing treatment inpolycondensation reaction, but by adding the swelling clay mineral to the reaction system after termination of the polycondensation reaction, thoroughly homogeneously mixing, and performing solid-liquid separation and drying, or by distilling off the solvent under reduced pressure.

In the method of the embodiment, a filler other than the swelling clay mineral treated with an organic compound may be used with the above swelling claymineral having been subj ected to organizing treatment, and the polycondensation reaction may be performed in the presence of them, or they may be added after termination of the polycondensation reaction, by which the filler is to be contained and well dispersed in a thermosetting resin composite material obtained.

As the filler other than the swelling clay minerals, at least one selected from calcium carbonate, barium sulfate, magnesia, alumina, zirconia, silica, aluminum powder, copper powder, zinc powder, graphite, molybdenum disulfide, and antimony sulfide is exemplified. It is preferred that the treatment of the filler with an organic compound be carried out by using, as the organic compound, an aliphatic or aromatic primary amine having about 10 to 35 carbon atoms or a silane coupling agent having a primary amine group on the terminals.

As the aliphatic or aromatic primary amines, e.g., n-dodecylamine, n-hexadecylamine, n-octadecylamine, n-nonadecylamine, p-tert-butylaniline, p-octylaniline, and p-dodecylaniline are exemplified, and as the silane coupling agents, e.g., 3-aminopropyltrimethoxysilane and 3-aminopropyltriethoxysilane are exemplified. Of these organic compounds, dodecylamine is especially preferred.

The treating methods of the fillers with the organic compounds are not especially restricted, and a method of treatment with the organic compounds in the state of melted liquids as they are, or a method of dissolving the organic compounds in an appropriate organic solvent and treating in the state of solutions may be used.

The kind and amount of a filler treated with an organic compound that is to be present in the reaction system or to be added to the reaction system after termination of the polycondensation reactionmaybe arbitrarily selected according to the use of the thermosetting resin composite material.

When bisphenol A as the compound having a phenolic hydroxyl group, and aniline as the primary amine are used respectively in the polycondensation reaction, a polybenz- oxazine resin represented by the following formula (I) is manufactured.

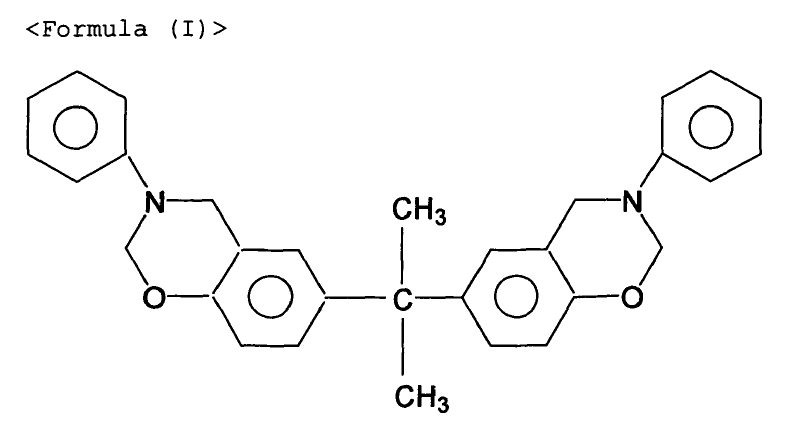

By heating at temperature of 120 to 300°C or so, the dihydrobenzoxazine ring of the polybenzoxazine resin opens to be self-crosslinked, or, when a crosslinking compound is present, the polybenzoxazine resin is cured by crosslinking the crosslinking compound at the same time with self-crosslinking. Accordingly, volatile by-products do not occur at the time of curing.

The thermosetting resin composite material obtained according to the method of the embodiment as described above is a material in which a swelling clay mineral such as montmorillonite is homogeneously dispersed and improved in strength and heat resistance, and preferably used for various uses, for example, friction materials, molding materials, machine parts, structural members, structural adhesives, and the like.

The embodiment also provides the thermosetting resin composite material obtained according to the method of the embodiment as described above and a friction material obtained by using the thermosetting resin composite material.

### Friction material:

The thermosetting resin composite material obtained by the method of the embodiment as described above is used in the friction material of the embodiment. A polybenzoxazine resin and a swelling clay mineral are contained in the composite material as essential components and, if desired, fillers other than the swelling clay mineral, e.g., various kinds of fillers, and a friction modifier are contained.

In the manufacture of the friction material, the thermosetting resin composite material may further contain, if necessary, thermosetting resins such as acondensedpolycyclic aromatic hydrocarbon resin to which a phenolic nucleus is introduced, and a novolak type phenol resin, various kinds of fibrous materials, cashew dust and rubber dust, by mixture with a mixer and the like.

When a condensed polycyclic aromatic hydrocarbon resin to which a phenolic nucleus is introduced and a novolak type phenol resin are present in thermosetting resin composite material, at the time of ring opening of the dihydrobenzoxazine ring and self-crosslinking of the polybenzoxazine resin by heating, the condensed polycyclic aromatic hydrocarbon resin and the novolak type phenol resin are crosslinked and cured as well.

As the fibrous materials, any of organic fibers and inorganic fibers may be used. As the organic fibers, highly strong aromatic polyamide fibers (Aramid fiber; trade name "Kevlar", manufactured by Du Pont-Toray Co., Ltd.), flame resisting acryl fibers, polyimide fibers, polyacrylate fibers, and polyester fibers may be exemplified. On the other hand, as the inorganic fibers, inorganic whiskers, e.g., potassium titanate whiskers and silicon carbide whiskers; glass fibers; carbon fibers; mineral fibers, e.g., wollastonite, sepiolite, attapulgite, halloysite, mordenite, and rock fiber; ceramic fibers, e.g., alumina silica fiber; and metal fibers, e.g., aluminum fiber, stainless steel fiber, copper fiber, brass fiber, and nickel fiber may be exemplified. These fibrous materials may be used by one kind alone, or two or more materials may be used in combination.

Inmanufacturing a friction material with the thermosetting resin composite material, for example, the thermosetting resin composite material is filled in a mold, preliminarily molded at ordinary temperature and pressure of from 5 to 30 MPa or so, and then heated and compression molded on the condition of a temperature of about 130 to 190°C and pressure of about 10 to 100 MPa for 5 to 35 minutes or so, and if necessary, heating treatment is performed at a temperature of about 160 to 270°C for 1 to 10 hours or so, whereby a desired friction material may be manufactured.

The thus-obtained friction material has excellent high temperature performance (reduction of wear loss).

### EXAMPLE

The embodiment will be described in further detail with reference to examples, but the invention is by no means restricted thereto.

Various performances are evaluated according to the following methods.
(1) Evaluation of widening of the distance between layers by montmorillonite (X-ray diffraction)
   (a) Pulverized and organized montmorillonite is filled in X-ray folder, and the range of 2θ = 3 to 10° are measured with an X-ray diffractometer ("XRD-6000", manufactured by Shimadzu Corporation).
   (b) A thermosetting resin composite material is made powder with a mortar, filled in an X-ray folder, and the layer-to-layer distance of montmorillonite in the composite material is measured with the X-ray diffractometer.
(2) Evaluation of dispersibility of montmorillonite (observation with a microscope)
   (a) A thermosetting resin composite material (1 g) is put on a glass Petri dish, melted in an oven, and observed with a microscope after cooling, and dispersibility of montmorillonite is evaluated according to the following criteria.
      A: Free of an agglomerate.
      B: A small agglomerate of a size of 20 µm or more is present. C: A large agglomerate of a size of 50 µm or more is present.
   (b) A thermosetting resin composite material is hardened at 250°C, and the state of dispersion of the montmorillonite in the hardened composite material is observed with a microscope, and dispersibility is evaluated according to the following criteria.
      A: Free of a block of agglomerate.
      B: A block of agglomerate is present.
(3) A heat resisting decomposition characteristic [measurement of TG-DTA (thermogravimetric variation)]
   A thermosetting resin composite materialor a thermosetting resin of a sample is thermoset with an electric oven at 180°C for 1 hour, and then heating treated at 250°C for 3 hours. The obtained resin after curing is pulverized with a sample mill to an average particle size of 50 µm and recovered to obtain a sample for TG-DTA measurement. The measurement is carried out on the condition of sample weight: 10 mg, temperature increasing rate: 10°C/min,measuringtemperature: 25 to 1,000°C, and measuring atmospheres: in atmospheric air with a measuring apparatus Max TG (manufactured by BRUKER JAPAN CO., LTD.) to find weight retention at 600°C.
(4) Frictional test by temperature
   Atestpiece is cut out of a friction material, and frictional test by temperature is performed with a frictional tester (a 1/10 scale tester) on the following condition, and wear losses (mm) of the test peace at every temperature are compared. Counterpart material: FC250
   Friction temperature: 100°C, 200°C, 300°C and 400°C
   Number of times of friction: 500 times
   Initial speed: 15 m/sec
   Deceleration: 0.3 G constant

### MANUFACTURE EXAMPLE 1

### Manufacture of montmorillonite to be organizing treated:

Montmorillonite (36 g) (Kunipia-G, manufactured by Kunimine Industries Co., Ltd.) and 720 g of distilled water are put in a beaker, stirred and mixed at 80°C, 3.6 g of aniline hydrochloride is added thereto and stirred and mixed at 80°C. Subsequently, the mixed solution is filtered, and a resulting cake is thoroughly washed with water and dried in an oven at 120°C to obtain 34 g of dried montmorillonite having been subjected to organizing treatment.

The dried montmorillonite having been subjected to organizing treatment is pulverized with a planetary ball mill. The widening of the distance between layers of the pulverized product is evaluated by X-ray diffraction according to the above (a) in evaluation test (1).

Further, for comparison, X-ray diffraction of untreated montmorillonite is performed similarly.

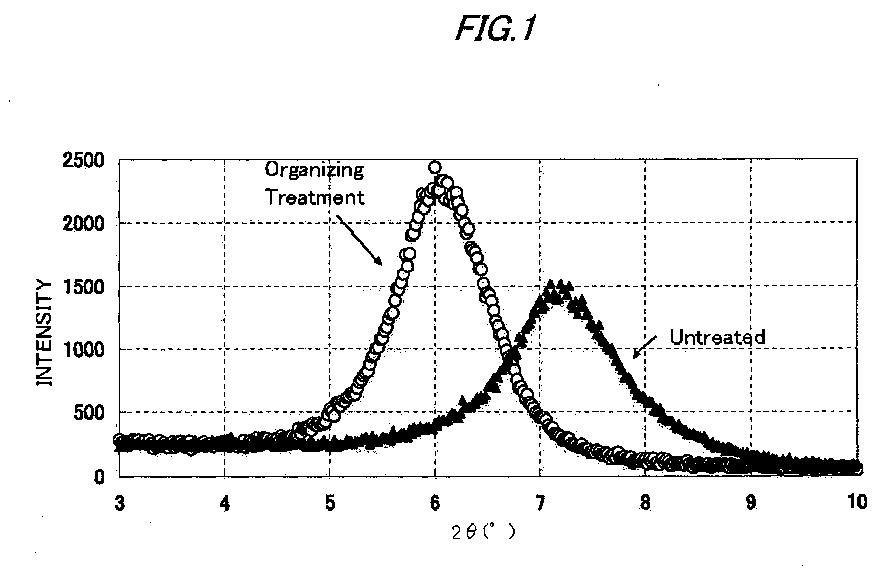

The results obtained are shown in Fig. 1. As a result of X-ray diffraction, it is confirmed that the distance between layers of the montmorillonite subjected to organizing treatment with aniline is extended.

### EXAMPLE 1

Bisphenol A (480 g), 390 g of aniline, 270 g of paraformaldehyde, 480 g of methyl ethyl ketone, and 55 g of the montmorillonite subjected to organizing treatment are put in a four-neck flask and stirred. After homogeneous mixing, the mixture is subjected to polycondensation reaction at 40°C for 1 hour, 50°C for 1 hour, and 80°C for 4 hours, respectively. After that, dehydration and desolvation are performed under reduced pressure of 0.06 MPa for 2 hours to obtain 1,005 g of a thermosetting resin composite material.

In connection with the obtained thermosetting resin composite material, dispersibility of montmorillonite is evaluated according to the above (a) in evaluation test (2), and also mass retention at 600°C is found according to evaluation test (3) for evaluation of heat resisting decomposition characteristic. The results obtained are shown in Table 1 below.

### EXAMPLE 2

Bisphenol A (480 g), 390 g of aniline, 270 g of paraformaldehyde, 480 g of methyl ethyl ketone, and 33 g of the montmorillonite subjected to organizing treatment are put in a four-neck flask and stirred. After homogeneous mixing, the mixture is subjected to polycondensation reaction at 40°C for 1 hour, 50°C for 1 hour, and 80°C for 4 hours, respectively. After that, dehydration and desolvation are performed under reduced pressure of 0.06 MPa for 2 hours to obtain 985 g of a thermosetting resin composite material.

In connection with the obtained thermosetting resin composite material, dispersibility of montmorillonite is evaluated according to the above (a) in evaluation test (2), and also mass retention at 600°C is found according to evaluation test (3) for evaluation of heat resisting decomposition characteristic. The results obtained are shown in Table 1 below.

### COMPARATIVE EXAMPLE 1

Bisphenol A (480 g), 390 g of aniline, 270 g of paraformaldehyde, and 480 g of methyl ethyl ketone are put in a four-neck flask and stirred. After homogeneous mixing, the mixture is subjected to polycondensation reaction at 40°C for 1 hour, 50°C for 1 hour, and 80°C for 4 hours, respectively. After that, dehydration and desolvation are performed under reduced pressure of 0.06 MPa for 2 hours to obtain 960 g of a thermosetting resin.

In connection with the obtained thermosetting resin, mass retention at 600°C is found according to evaluation test (3) for evaluation of heat resisting decomposition characteristic. The results obtained are shown in Table 1 below.

### COMPARATIVE EXAMPLE 2

Bisphenol A (480 g), 390 g of aniline, 270 g of paraformaldehyde, and 480 g of methyl ethyl ketone are put in a four-neck flask and stirred. After homogeneous mixing, the mixture is subjected to polycondensation reaction at 40°C for 1 hour, 50°C for 1 hour, and 80°C for 4 hours, respectively. After that, dehydration and desolvation are performed under reduced pressure of 0.06 MPa for 2 hours to obtain 960 g of a thermosetting resin.

To 950 g of the thermosetting resin is added 50 g of montmorillonite having been subjected to organizing treatment obtained in Manufacture Example 1, and mixed with a biaxial kneading extruder to obtain a thermosetting resin composite material.

In connection with the obtained thermosetting resin composite material, dispersibility of montmorillonite is evaluated according to the above (a) in evaluation test (2), and also mass retention at 600°C is found according to evaluation test (3) for evaluation of heat resisting decomposition characteristic. The results obtained are shown in Table 1 below.

**TABLE 1**

| | Example 1 | Example 2 | Comparative Example 1 | Comparative Example 2 |
|---|---|---|---|---|
| Dispersibility of montmorillonite | A | A | - | B |
| Heat resisting decomposition characteristic (%) (weight retention at 600°C) | 37 | 33 | 31 | 35 |

From the results in Table 1, in Examples 1 and 2 wherein polycondensation reaction is performed in the presence of montmorillonite having been subjected to organizing treatment, the dispersibility of montmorillonite is good as compared with Comparative Example 2 wherein montmorillonite subjected to organizing treatment isblendedwithabiaxial kneading extruder.

With respect to a heat resisting decomposition characteristic (mass retention at 600°C), it can be seen that the sample in Example 1 is improved as compared with the sample in Comparative Example 2 containing the same amount of montmorillonite having been subjected to organizing treatment. Further, it is known that the sample in Example 2 is improved in a heat resisting decomposition characteristic as compared with the sample in Comparative Example 1 not containing montmorillonite.

### EXAMPLES 3 AND 4 AND COMPARATIVE EXAMPLES 3 AND 4

Materials for molding each having the blending composition shown in Table 2 below are prepared with a mixer by using the thermosetting resin composite materials obtained in Examples 1 and 2, the thermosetting resin obtained in Comparative Example 1 and the thermosetting resin composite material obtained in Comparative Example 2. For increasing curing speed, 30 wt% of 8 weight parts of the resin used is straight novolak resin.

Each of the materials for molding is preliminarily molded on the condition of ordinary temperature and pressure of 30 MPa, and then heat compression molded by hot press on the condition of temperature of 180°C, pressure of 30 MPa for 10 minutes, followed by heat treatment of the obtained molded product at 250°C for 3 hours to obtain each friction material.

In connection with the friction materials, wear loss is measured by frictional test by temperature according to evaluation test (4). The results obtained are shown in Table 3 below.

**TABLE 2**

| | |
|---|---|
| Blending Composition of Materials for Forming a Friction Material | |

| Name of Material | Weight Parts |
|---|---|
| Composite material or resin | 8 |
| Rubber dust | 8 |
| Barium sulfate | 36.5 |
| Zirconia | 2 |
| Scaly graphite | 5.5 |
| Aramid pulp | 4 |
| Potassium Titanate | 23 |
| Copper fiber | 13 |

**TABLE 3**

| | | Example 3 | Example 4 | Comparative Example 3 | Comparative Example 4 |
|---|---|---|---|---|---|
| The kind of composite material or resin | | Example 1 | Example 2 | Comparative Example 1 | Comparative Example 2 |
| Wear loss (mm) | 100°C | 0.04 | 0.04 | 0.04 | 0.04 |
| | 200°C | 0.06 | 0.06 | 0.06 | 0.05 |
| | 300°C | 0.10 | 0.11 | 0.14 | 0.12 |
| | 400°C | 0.19 | 0.22 | 0.27 | 0.21 |

Wear loss: measured with a 1/10 scale tester.

From the results in Table 3, it is seen that the friction material in Example 3 using the thermosetting resin composite material of Example 1 is improved in wear resistance at high temperature as compared with the friction material in Comparative Example 4 using the thermosetting resin composite material of Comparative Example 2 containing the same amount of montmorillonite having been subjected to organizing treatment. Further, it is understood that the friction material in Example 4 using the thermosetting resin composite material of Example 2 is improved in wear resistance at high temperature as compared with the friction material in Comparative Example 3 using the thermosetting resin of Comparative Example 1 not containing montmorillonite.

### EXAMPLE 5

Bisphenol A (480 g), 390 g of aniline, 270 g of paraformaldehyde, and 480 g of toluene are put in a four-neck flask and stirred. After homogeneous mixing, the mixture is subjected to polycondensation reaction at 40°C for 1 hour, 50°C for 1 hour, and 80°C for 4 hours, respectively, and after that, 103 g of montmorillonite having been subjected to organizing treatment (trade name "S-Ben NX", manufactured by HOJUN, a product treated with dimethyldioctadecylammonium chloride) is added. After stirring and mixing the reaction mixture at 80°C for 1 hour, dehydration and desolvation are performed under reduced pressure of 0.06 MPa for 2 hours to obtain a thermosetting resin composite material.

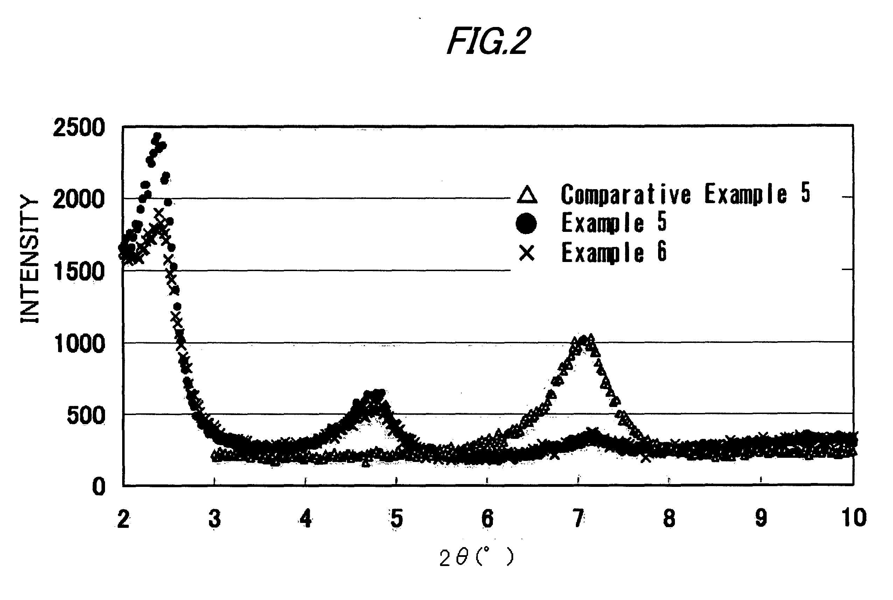

With respect to the thermosetting resin composite material, the distance between layers of inontmorillonite in the composite material is measured according to the above (b) in evaluation test (1). The results obtained are shown in Fig. 2.

The dispersibility of the montmorillonite is evaluated according to the above (b) in evaluation test (2). The results obtained are shown in Table 4 below.

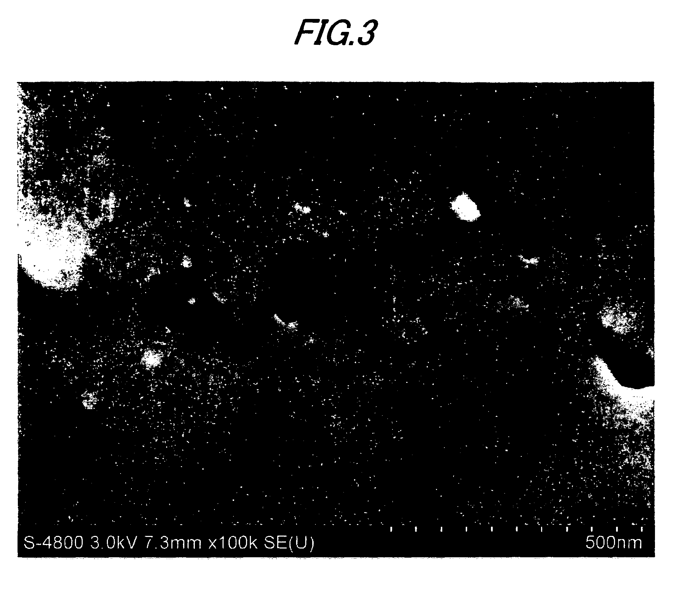

The microphotograph of the thermosetting resin composite material is shown in Fig. 3.

### EXAMPLE 6

A thermosetting resin composite material is prepared in the same manner as in Example 5 except for using 88 g of "Esben N012S" (a product treated with oleyl-bis(2-hydroxyethyl)-methylammonium chloride, manufactured by HOJUN) as the montmorillonite having been subjected to organizing treatment in place of "Esben NX". The thermosetting resin composite material is evaluated in the same manner as in Example 5. The results obtained are shown in Fig. 2 and Table 4.

### COMPARATIVE EXAMPLE 5

A thermosetting resin composite material is prepared in the same manner as in Example 5 except for using 60 g of untreated montmorillonite (trade name "Kunipia-G", manufactured by Kunimine Industries Co., Ltd.) in place of "Esben NX". The thermosetting resin composite material is evaluated in the same manner as in Example 5. The results obtained are shown in Fig. 2 and Table 4.

**TABLE 4**

| | | | |
|---|---|---|---|
| | Example 5 | Example 6 | Comparative Example 5 |
| Dispersibility of montmorillonite | A | A | B |

It is seen from the results of X-ray diffraction that, as shown in Fig. 2, in Examples 5 and 6, wherein montmorillonite having been subjected to organizing treatment is added after the polycondensation reaction, the distance between layers of montmorillonite widens as compared with the sample in Comparative Example 5 wherein untreated montmorillonite is added after the polycondensation reaction.

With respect to dispersibility of the montmorillonite, a block of agglomerate is not seen and good dispersibility is revealed in Examples 5 and 6, while a block of agglomerate is present and dispersibility is not good in Comparative Example 5.

### EXAMPLES 7 AND 8 AND COMPARATIVE EXAMPLE 6

Materials for molding each having the blending composition shown in Table 2 above are prepared with a mixer by using the thermosetting resin composite materials obtained in Examples 5 and 6 and Comparative Example 5. Of 8 weight parts of the resin used, 30 wt% is straight novolak resin.

Each of the materials for molding is preliminarily molded on the condition of ordinary temperature and pressure of 30 MPa, and thenheat compression molded by hot press on the condition of temperature of 180°C, pressure of 30 MPa for 10 minutes, followed by heat treatment of the obtained molded product at 250°C for 3 hours to obtain each friction material.

In connection with the friction materials, wear loss is measured by frictional test by temperature according to evaluation test (4). The results obtained are shown in Table 5 below.

**TABLE 5**

| | | Example 7 | Example 8 | Comparative Example 6 |
|---|---|---|---|---|
| The kind of composite material | | Example 5 | Example 6 | Comparative Example 5 |
| Wear loss (mm) | 100°C | 0.04 | 0.04 | 0.04 |
| | 200°C | 0.06 | 0.06 | 0.08 |
| | 300°C | 0.10 | 0.13 | 0.19 |
| | 400°C | 0.18 | 0.21 | 0.25 |

Wear loss: measured with a 1/10 scale tester.

From the results in Table 5, it is seen that the friction materials in Examples 7 and 8 using the thermosetting resin composite materials containing the montmorillonites having been subjected to organizing treatment in Examples 5 and 6, wear resistance at 300°C and 400°C is improved as compared with the friction material in Comparative Example 6 using the thermosetting resin composite material containing the untreated montmorillonite in Comparative Example 5.

According to the manufacturing method of a thermosetting resin composite material of the invention, a thermosetting resin composite material in which a swelling clay mineral such as montmorillonite is homogeneously dispersed and heightened in strength and heat resistance can be manufactured in high efficiency. The thermosetting resin composite material can provide a friction material improved in high temperature performances.

While description has been made in connection with specific embodiment and examples of the present invention, it will be obvious to those skilled in the art that various changes and modification may be made therein without departing from the present invention. It is aimed, therefore, to cover in the appended claims all such changes and modifications falling within the true spirit and scope of the present invention.

## Claims

1. A method for manufacturing a thermosetting resin composite material, the method comprising:
forming a polybenzoxazine resin by polycondensation reaction of a compound having a phenolic hydroxyl group, primary amines, and formaldehydes,
wherein the polycondensation reaction is performed in a presence of a swelling clay mineral subjected to organizing treatment, or the swelling clay mineral is added to the reaction system after a termination of the polycondensation reaction.

2. The method according to claim 1, wherein the swelling clay mineral subjected comprises montmorillonite which is subjected to the organizing treatment.

3. The method according to claim 1, wherein the organic treatment comprises a treatment with at least one of amines and quaternary ammonium salt.

4. The method according to claim 3, wherein the amines comprise aromatic amines.

5. The method according to claim 1, wherein the primary amines comprise primary aromatic amines.

6. A thermosetting resin composite material that is obtained by the method according to claim 1.

7. A friction material comprising the thermosetting resin composite material according to claim 6.
